# FASCICULE DE BREVET EUROPEEN

(11) **EP 2 889 048 B1**
(45) Date de publication et mention de la délivrance du brevet: **10.11.2021**
(21) Numéro de dépôt: 14200215.3
(22) Date de dépôt: 23.12.2014
(51) Int. Cl.: A61M 1/02

(54) **Procédé d'extraction de composants sanguins à l'aide d'une poche filtrante**
Verfahren zur Extraktion von Blutbestandteilen mithilfe einer Filtertasche
Method for extracting blood components by means of a filter bag

(30) Priorité: 26.12.2013 FR 1363617
(43) Date de publication de la demande: 01.07.2015
(73) Titulaire: MACO PHARMA, 59420 Mouvaux (FR)
(72) Inventeur: Deverre, Frédéric, 34090 Montpellier (FR); Chavatte, Arnaud, 62350 Saint Floris (FR); Moisan, Kevin, 59200 Tourcoing (FR)
(74) Mandataire: Sayettat, Julien Christian

(56) Documents cités:
- EP-A1- 0 600 804
- EP-A1- 0 953 361
- WO-A1-02/43790
- US-A1- 2003 195 104

## Description

L'invention concerne un procédé pour extraire un premier et un deuxième composant sanguin contenu dans une poche primaire d'un système à poches, ainsi qu'une poche filtrante pour la mise en œuvre de ce procédé, et un appareil pour mettre en œuvre un tel procédé.

Le document EP 0 953 361 A1 est considéré comme représentant l'art antérieur le plus proche et divulgue le préambule de la revendication 1. Le document WO 02/43790 A1 divulgue une poche filtrante selon l'art antérieur.

L'invention s'applique au domaine de la transfusion sanguine, en particulier au traitement du sang, et notamment la séparation des composants sanguins.

Le sang total est constitué de deux types de composants : les cellules sanguines comprenant les globules rouges, les leucocytes et les plaquettes, et le plasma dans lequel les cellules sanguines sont en suspension. Actuellement, ne sont transfusés que les composants sanguins nécessaires aux patients. Aujourd'hui, dans les centres de transfusion ou les hôpitaux, ces différents composants sanguins sont séparés par centrifugation : le sang total d'un donneur est recueilli dans une poche dite poche primaire d'un système à poches. Puis, le système à poches est placé dans un insert plastique, lui-même disposé dans un godet métallique d'une centrifugeuse afin de séparer les différents composants sous l'effet de la force centrifuge.

Il existe deux types de centrifugation. La centrifugation dite douce du sang total conduit à le séparer en deux couches : une couche inférieure riche en globules rouges appelée concentré de globules rouges (CGR) ; et une couche supérieure contenant le plasma, les plaquettes et les leucocytes appelée plasma riche en plaquettes (PRP). La centrifugation dite dure conduit à une séparation en trois couches : une couche inférieure de CGR ; une couche supérieure de plasma pauvre en plaquettes (PPP) ; et une couche intermédiaire formée essentiellement de leucocytes et de plaquettes, dénommée couche leuco-plaquettaire ou buffy coat (BC).

Ces composants sanguins ainsi séparés sont notamment destinés à être transfusés à un patient qui en a besoin. Lors de cette transfusion, il est bien connu que les leucocytes sont indésirables en ce qu'ils sont susceptibles de provoquer chez le patient des réactions gênantes et/ou potentiellement dangereuses.

Il en est de même pour certaines substances tel que le prion, l'agent responsable des encéphalopathies spongiformes transmissibles, notamment de la variante de la maladie de Creutzfeldt-Jakob chez l'homme puisque des études ont montré qu'il existe un risque probable de transmission du prion lors de transfusions sanguines.

Pour éliminer ces substances indésirables, on connaît par exemple du document EP-A-526 678, des unités de filtration qui comprennent une enveloppe extérieure souple renfermant un milieu filtrant et munie d'au moins un port d'entrée et d'au moins un port de sortie entre lesquels le fluide à filtrer s'écoule suivant une direction. Le milieu filtrant délimite avec l'enveloppe extérieure un compartiment d'entrée pour le fluide à filtrer et un compartiment de sortie pour le filtrat.

Ces unités de filtration constituent des éléments supplémentaires du système à poches qui complexifient non seulement la fabrication mais aussi l'utilisation de tels systèmes. En outre, de tels systèmes à poches sont encombrants et relativement difficiles à mettre en place dans l'insert plastique en vue de la centrifugation.

L'invention propose un procédé pour extraire des composants sanguins à l'aide d'un système à poches simplifié. Le système à poches comprend notamment une poche filtrante permettant à la fois de filtrer un composant sanguin et d'en stocker un autre.

A cet effet et selon un premier aspect, l'invention propose un procédé pour extraire au moins un premier et un deuxième composant sanguin contenus dans une poche primaire d'un système à poches, ledit système à poches comprenant en outre au moins une poche satellite de recueil du premier composant sanguin en communication fluidique avec la poche primaire, et une poche filtrante disposée sur le chemin d'écoulement défini entre la poche primaire et la poche satellite de recueil du premier composant sanguin, la poche filtrante comprenant un compartiment de filtration disposant d'un milieu filtrant et un compartiment de stockage du deuxième composant sanguin, lesdits compartiments communiquant entre eux par un passage pouvant être fermé, ledit procédé comprenant les étapes de :
- compresser la poche primaire de sorte à extraire le premier composant sanguin de la poche primaire et à le transférer au moins en partie dans la poche satellite de recueil du premier composant sanguin en passant par ladite poche filtrante,
- poursuivre la compression de la poche primaire de sorte à extraire le deuxième composant sanguin de la poche primaire et à le transférer au moins en partie dans le compartiment de stockage de ladite poche filtrante, et
- fermer le passage entre le compartiment de stockage et le compartiment de filtration.

Selon un deuxième aspect, l'invention concerne une poche filtrante pour la mise en œuvre du procédé selon le premier aspect de l'invention, ladite poche filtrante comprenant une première et deuxième feuille en matière thermoplastique souple solidarisées l'une à l'autre sur leur périphérie de sorte à former un volume intérieur, au moins un orifice d'entrée et un orifice de sortie, et un cordon de soudure entre les feuilles agencé pour former le compartiment de filtration et le compartiment de stockage, ledit cordon de soudure comprenant une partie horizontale et une partie verticale agencée pour former avec un bord de la poche filtrante un canal en communication fluidique avec l'un des orifices d'entrée ou sortie de la poche filtrante.

Selon un troisième aspect, l'invention porte sur un appareil pour mettre en œuvre le procédé selon le premier aspect de l'invention, ledit appareil comprenant :
- un moyen de compression de la poche primaire de sorte à extraire au moins un composant sanguin, et
- un dispositif de contrôle destiné à contrôler l'écoulement des fluides dans le système à poches, ledit dispositif de contrôle comprenant au moins un clamp pour fermer le passage entre le compartiment de stockage et le compartiment de filtration.

D'autres objets et avantages apparaîtront au cours de la description qui suit.
Les figures 1 et 2 représentent une vue schématique d'un système à poches pour la séparation des composants sanguins comprenant une poche satellite de recueil d'un premier composant sanguin et une poche satellite de recueil du deuxième composant sanguin en dérivation et en série, respectivement.
La figure 3 représente une vue schématique d'un système à poches pour la séparation des composants sanguins comprenant une poche filtrante.
La figure 4 représente une vue schématique d'une variante de réalisation d'une poche filtrante.
La figure 5 représente une vue schématique et en éclaté d'une autre variante de réalisation d'une poche filtrante.
La figure 6 représente une vue schématique et en perspective d'un réceptacle destiné à recevoir un système à poches.
La figure 7 représente une vue schématique et en perspective du réceptacle de la figure 6 sans plaque de pressage.
La figure 8 représente une vue schématique et en perspective d'une plaque de pressage destinée à être reçue dans le réceptacle de la figure 7.
La figure 9 représente une vue schématique et en perspective du réceptacle de la figure 6 dans lequel une poche primaire, une poche filtrante et des portions de tubulures sont disposées.
La figure 10 représente de façon schématique un appareil pour l'extraction de composants sanguin.
La figure 11 représente de façon schématique un moyen de réception du réceptacle de l'appareil de la figure 10.
La figure 12 représente de façon schématique le dispositif de contrôle d'un appareil pour extraire les composants sanguins en relation avec le système à poches de la figure 3.

Lors d'un don de sang, le sang est généralement recueilli dans un système à poches comprenant une poche primaire destiné à recueillir le sang prélevé d'un donneur, et une ou plusieurs autres poches satellites pour le recueil des composants sanguins séparés par centrifugation, lesdites poches étant associées entre elles par des tubulures.

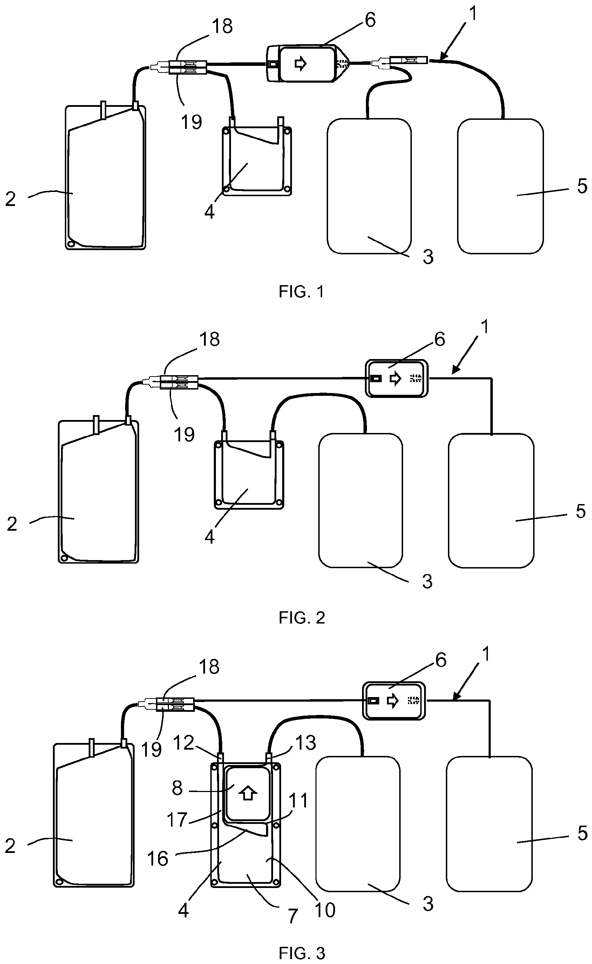

Des exemples particuliers d'un tel système à poches sont représentés sur les figures 1 à 3. Sur ces figures, le système à poches 1 comprend une poche primaire 2 destinée à contenir le sang recueilli d'un donneur.

Une fois le sang recueilli dans cette poche primaire et après centrifugation du système à poches, cette poche primaire 2 comprend au moins un premier composant sanguin et un deuxième composant sanguin. Par exemple, la poche primaire 2 contient un composant plasmatique riche en plaquettes et un composant globulaire. En variante, la poche primaire 2 contient trois composants sanguins qui sont un composant plasmatique, un composant leuco-plaquettaire et un composant globulaire.

Dans la position normale d'utilisation de la poche 2, le composant plasmatique se situe dans le haut de ladite poche, le composant globulaire dans le bas de la poche 2 et le composant leuco-plaquettaire entre le composant plasmatique et le composant plaquettaire.

Le système à poches 1 comprend en outre au moins une poche satellite 3 de recueil du premier composant sanguin en communication fluidique avec la poche primaire 2, et une poche satellite 4 de recueil du deuxième composant sanguin en communication fluidique avec la poche primaire 2.

Sur la figure 1, la poche satellite 3 de recueil du premier composant sanguin et la poche satellite 4 de recueil du deuxième composant sanguin sont disposées en parallèle ou en dérivation, c'est-à-dire que ces poches sont reliées par des tubulures différentes à la poche primaire 2 par l'intermédiaire d'un connecteur à trois branches.

Sur les figures 2 et 3, la poche satellite 4 de recueil du deuxième composant sanguin est disposée sur le chemin d'écoulement défini entre la poche primaire 2 et la poche satellite 3 de recueil du premier composant sanguin. La poche satellite 3 de recueil du premier composant sanguin et celle de recueil du deuxième composant sanguin sont donc disposées en série.

Sur les figures 1 à 3, le système à poches comprend en outre une poche satellite 5 de recueil d'un troisième composant sanguin contenu dans la poche primaire 2, ladite poche satellite 5 étant en communication fluidique avec la poche primaire 2.

Cette poche satellite 5 de recueil d'un troisième composant sanguin est notamment destinée à contenir un composant globulaire et contient avantageusement une solution additive de conservation des globules rouges, telle que par exemple une solution SAGM (saline - glucose - adénine - mannitol).

En particulier, les poches 2,3,4,5 du système à poches 1 sont reliées entre elles par des tubulures souples et sécables.

Dans une réalisation particulière représentée sur les figures 1 à 3, le système à poches 1 comprend au moins une unité de filtration 6 destinée notamment à filtrer un composant sanguin. En particulier, l'unité de filtration 6 permet d'éliminer les leucocytes du composant sanguin.

Par exemple, sur les figures 1 à 3, le système à poches comprend une unité de filtration 6 destinée à filtrer le troisième composant sanguin, tel que le composant globulaire. Cette unité de filtration 6 est disposée sur le chemin d'écoulement défini entre la poche primaire 2 et la poche satellite 5 de recueil du troisième composant sanguin.

Sur la figure 1, l'unité de filtration 6 est disposée non seulement sur le chemin d'écoulement défini entre la poche primaire 2 et la poche satellite 5 de recueil du troisième composant sanguin mais aussi sur le chemin d'écoulement défini entre la poche primaire 2 et la poche satellite 3 de recueil du premier composant sanguin. Ainsi, l'unité de filtration 6 permet d'éliminer de façon séquentielle les leucocytes du composant plasmatique et du composant globulaire.

En variante, le système à poches 1 comprend une autre unité de filtration destinée notamment à filtrer le premier composant sanguin, tel que le composant plasmatique. Cette unité de filtration est disposée sur le chemin d'écoulement défini entre la poche primaire 2 et la poche satellite 3 de recueil du premier composant sanguin.

Un exemple particulier de système à poches selon l'invention est représenté sur la figure 3. Dans cet exemple, la poche satellite 4 de recueil du deuxième composant sanguin inclut une unité de filtration du premier composant sanguin de sorte à former une poche filtrante 7 disposée sur le chemin d'écoulement défini entre la poche primaire 2 et la poche satellite 3 de recueil du premier composant sanguin.

En relation avec les figures 3 à 5, la poche filtrante 7 comprend un compartiment de filtration 8 disposant d'un milieu filtrant 9 et un compartiment de stockage 10 du deuxième composant sanguin, lesdits compartiments 8,10 communiquant entre eux par un passage 11 pouvant être fermé.

La poche 7 est pourvue d'un orifice d'entrée 12 et d'un orifice de sortie 13. En particulier, l'orifice d'entrée 12 est en communication avec le compartiment de stockage 10 et l'orifice de sortie 13 est en communication avec le compartiment de filtration 8.

Le milieu filtrant 9 est notamment un milieu pour éliminer les leucocytes du sang, tel que par exemple le milieu filtrant décrit dans le document EP-0 953 361 ou EP-1 336 417.

Le milieu filtrant 9 définit dans le compartiment de filtration 8 une chambre d'entrée du composant à filtrer et une chambre de sortie du composant filtré.

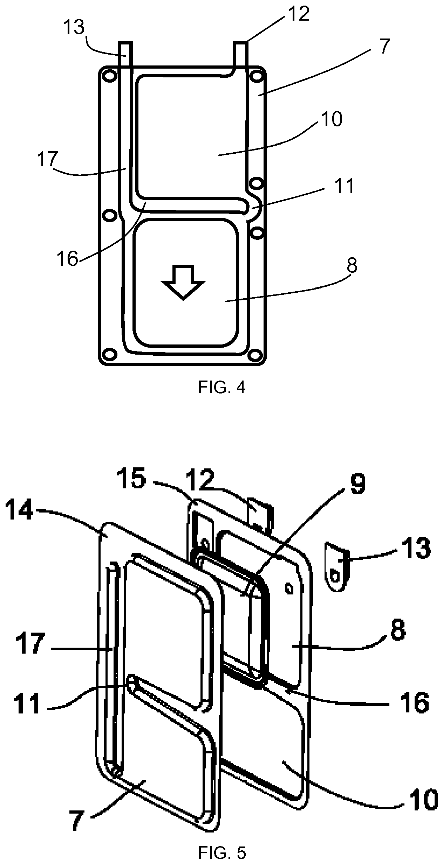

En particulier et comme montré sur la figure 5, la poche filtrante 7 comprend une première et deuxième feuille 14,15 en matière thermoplastique souple solidarisées l'une à l'autre sur leur périphérie de sorte à former un volume intérieur, ladite poche comportant un cordon de soudure 16 entre les feuilles, agencé pour former le compartiment de filtration 8 et le compartiment de stockage 10, ledit cordon de soudure 16 étant agencé pour former ledit passage 11 entre les compartiments 8,10.

Selon une réalisation, le passage 11 entre le compartiment de filtration 8 et le compartiment de stockage 10 est fermé par soudage des deux feuilles 14,15.

En relation avec les figures 3 à 5, les orifices 12,13 d'entrée et de sortie de la poche filtrante 7 sont disposés au voisinage d'un bord supérieur de la poche filtrante 7.

Cette disposition particulière des orifices d'entrée et de sortie permet d'éviter l'écrasement des tubulures reliant les différentes poches lorsque le système à poches 1 est disposé dans un insert plastique en vue de sa centrifugation ou dans un réceptacle comme décrit ci-après.

Comme illustré sur les figures 3 et 4, les orifices 12,13 d'entrée et/ou de sortie sont formés par des portions de tubulures soudées entre les feuilles 14, 15 formant la poche filtrante 7.

En variante et comme représenté sur la figure 5, les orifices 12,13 d'entrée et/ou de sortie sont formés de pièces moulées par injection, soudées sur la surface extérieure d'une ou de chacune des feuilles 14, 15 formant la poche filtrante 7.

Dans le cas où les orifices 12,13 d'entrée et/ou sortie sont disposés sur un même bord de la poche filtrante 7, le cordon de soudure 16 définissant le compartiment de filtration 8 et le compartiment de stockage 10 comprend une partie horizontale et une partie verticale qui est agencée pour former avec un bord de la poche filtrante 7, un canal 17 en communication fluidique avec l'un des orifices 12,13 d'entrée ou sortie de la poche filtrante 7.

Notamment, le compartiment de filtration 8 est disposé au dessus de la partie horizontale du cordon de soudure 16 de la poche filtrante. Ainsi, le compartiment de filtration 8 se situe au dessus du compartiment de stockage 10.

Par la suite, les termes "dessus", "dessous", "vertical", "horizontal" sont définis par rapport à la poche filtrante 7 dans sa position normale d'utilisation lors de l'extraction des composants sanguins, c'est-à-dire lorsqu'elle est placée verticalement, orifices d'entrée 12 / sortie 13 placés en haut.

Dans la configuration de la figures 5 et 3, l'orifice d'entrée 12 débouche dans le canal 17 formé par le cordon de soudure 16. Le composant sanguin qui traverse cette poche filtrante 7 s'écoule d'abord dans ce canal 17, traverse le compartiment de stockage 10, le passage 11 entre le compartiment de stockage 10 et le compartiment de filtration 8, puis traverse le compartiment de filtration 8 et ressort par l'orifice de sortie 13.

Dans la configuration de la figure 4, l'orifice de sortie 13 débouche dans le canal 17. Dans cette configuration, le composant sanguin qui traverse cette poche filtrante 7 entre d'abord par l'orifice d'entrée 12, traverse le compartiment de stockage 10, le passage 11 entre le compartiment de stockage 10 et le compartiment de filtration 8, puis traverse le compartiment de filtration 8, s'écoule dans le canal 17 et ressort par l'orifice de sortie 13.

La chambre d'entrée du compartiment de filtration 8 est en communication avec le passage 11 de la poche filtrante 7 et la chambre de sortie du compartiment de filtration 8 est en communication avec l'orifice 13 de sortie de la poche filtrante 7.

Dans une réalisation particulière non représentée, le cordon de soudure 16 comprend une partie fragile de sorte à pouvoir séparer le compartiment de filtration 8 et le compartiment de stockage 10. La séparation des compartiments 8,10 est réalisée après le recueil du deuxième composant sanguin dans le compartiment 10 dédié de la poche filtrante 7 et après fermeture du passage 11.

Par exemple, la partie fragile est constituée par une ligne de découpe comprenant des perforations.

Ce compartiment de stockage 10 séparé est ensuite stocké et/ou utilisé afin de réaliser un pool de deuxièmes composants sanguins. Le compartiment de filtration 8 peut être jeté.

Pour réaliser le pool de deuxièmes composants sanguins, la poche filtrante 7, dépourvue ou non de son compartiment de filtration 8 et dans laquelle est stockée le deuxième composant sanguin, est séparée du reste du système à poches 1. Entre quatre et sept poches filtrantes 7 sont ensuite connectées de façon stérile à un système de pool de buffy-coat pour assembler mélanger leur contenu dans une poche de transfert.

Lors de cette opération d'assemblage, le deuxième composant sanguin s'écoule dans la poche de transfert via l'orifice d'entrée 12 de chaque poche filtrante. Dans une réalisation particulière, le cordon de soudure 16 est agencée de sorte à faciliter l'écoulement du deuxième composant sanguin en dehors du compartiment de stockage via l'orifice d'entrée 12.

En relation avec les figures 3 et 5, la partie horizontale du cordon de soudure 16 présente une épaisseur décroissante en direction de l'orifice 12 d'entrée de la poche filtrante, de sorte à créer une pente facilitant l'écoulement du deuxième composant en dehors de son compartiment de stockage 10.

Afin de contrôler l'écoulement des fluides dans le système à poches 1, certaines des tubulures et/ou des orifices d'entrée et sortie des poches comprennent un élément de fermeture comportant une zone de fragilité pouvant être rompue pour permettre l'écoulement de fluide.

Sur les figures 1 à 3, la tubulure reliant la poche primaire 2 à la poche satellite 4 de recueil du deuxième composant sanguin et la tubulure reliant la poche primaire 2 à la poche satellite 5 de recueil du troisième composant sanguin sont pourvues chacune d'un tel élément de fermeture 18,19.

Pour extraire au moins un premier composant sanguin contenu dans la poche primaire 2 d'un système à poches 1 tel que décrit ci-dessus en relation avec les figures 1 à 3, on compresse la poche primaire 2 de sorte à le transférer dans la poche satellite 3 de recueil du composant.

Quand la poche primaire contient un deuxième et un troisième composant sanguin, en poursuivant la compression de la poche primaire 2, il est possible de transférer le deuxième composant sanguin dans une poche satellite 4 de recueil du deuxième composant sanguin et le troisième composant sanguin dans une poche satellite 5 de recueil du troisième composant sanguin.

Un premier exemple particulier de procédé pour extraire au moins un premier et deuxième composant contenus dans la poche primaire 2 d'un système à poches comme illustré sur la figure 2 est maintenant décrit ci-après.

Dans ce procédé, le système à poches comprend en outre au moins une poche satellite 3 de recueil du premier composant sanguin en communication fluidique avec la poche primaire 2, et une poche satellite 4 de recueil du deuxième composant sanguin disposée sur le chemin d'écoulement défini entre la poche primaire 2 et la poche satellite 3 de recueil du premier composant sanguin.

Le premier composant sanguin est par exemple un composant plasmatique et le deuxième composant sanguin, un composant leuco-plaquettaire.

Les poches satellites 3,4 de recueil du premier et deuxième composant sanguin sont ainsi disposées en série.

Ledit procédé de cet exemple comprend les étapes de :
- contraindre la poche satellite 4 de recueil du deuxième composant sanguin selon un premier volume,
- compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- après l'extraction du premier composant sanguin de la poche primaire 2, contraindre la poche satellite 4 de recueil du deuxième composant sanguin selon un deuxième volume,
- poursuivre la compression de la poche primaire 2 de sorte à extraire le deuxième composant sanguin de la poche primaire 2 et le transférer au moins en partie dans la poche satellite 4 de recueil du deuxième composant sanguin contrainte selon le deuxième volume.

La contrainte appliquée sur la poche satellite 4 de recueil du deuxième composant sanguin évite le gonflement de cette poche lorsque celle-ci reçoit le premier composant sanguin et permet donc un transfert plus rapide du premier composant sanguin dans la poche satellite 3 dédiée.

Le premier volume de contrainte de la poche satellite 4 de recueil du deuxième composant sanguin est ainsi choisi de sorte à permettre un écoulement rapide du composant sanguin au travers de cette poche satellite 4. Il est donc minimal, par exemple inférieur à 20 ml.

Encore plus avantageusement, le premier volume de contrainte est déterminé en fonction du volume de premier composant sanguin que l'on souhaite obtenir dans le volume de deuxième composant sanguin.

En particulier, dans le cas d'un composant leuco-plaquettaire, il est recommandé d'avoir un volume de composant plasmatique d'environ 20 à 40 ml dans le composant leuco-plaquettaire final afin de mettre les plaquettes en suspension. Ainsi, le premier volume de contrainte de la poche satellite 4 de recueil du deuxième composant sanguin est compris entre 20 et 40 ml, particulièrement entre 25 et 35 ml.

Après le transfert d'au moins une partie du premier composant sanguin dans la poche satellite 3 dédiée, la poche satellite 4 de recueil du deuxième composant sanguin est contrainte selon un deuxième volume. Notamment, le deuxième volume est supérieur au premier volume.

Ce deuxième volume correspond au volume de deuxième composant sanguin, notamment le volume final de composant leuco-plaquettaire, que l'on souhaite obtenir. Par exemple, le deuxième volume de contrainte est compris entre 40 et 80 ml, notamment entre 50 et 60 ml.

Ce procédé permet donc de maîtriser à la fois le volume final de composant leuco-plaquettaire et le volume de composant plasmatique diluant le composant leuco-plaquettaire.

Dans un mode de réalisation particulier, après transfert d'au moins une partie du premier composant sanguin dans la poche satellite 3 de recueil du premier composant sanguin, le procédé comprend l'étape de couper la communication fluidique entre la poche satellite 3 de recueil du premier composant sanguin et la poche satellite 4 de recueil du deuxième composant sanguin contrainte selon le premier volume.

Le transfert du premier composant sanguin est suivi à l'aide d'un détecteur optique placé au niveau de la poche primaire 2 ou de la poche satellite 4 de recueil du deuxième composant sanguin ou encore de la tubulure entre la poche primaire 2 et ladite poche satellite 4.

Lorsque l'interface entre le premier composant sanguin et le deuxième composant sanguin est détecté par ce détecteur, on ferme la communication fluidique entre la poche satellite 3 de recueil du premier composant sanguin et la poche satellite 4 de recueil du deuxième composant sanguin.

Lorsque le système à poches 1 comprend une poche satellite 5 de recueil d'un troisième composant sanguin contenu dans la poche primaire 2, ladite poche satellite étant en communication fluidique avec la poche primaire, ledit procédé comprend en outre l'étape de :
- après l'extraction du deuxième composant sanguin de la poche primaire 2, poursuivre la compression de la poche primaire de sorte à extraire le troisième composant sanguin de la poche primaire 2 et le transférer dans la poche satellite 5 de recueil du troisième composant sanguin.

Le troisième composant sanguin est par exemple un composant globulaire.

Dans le cas d'un système à poches selon l'invention comprenant une poche filtrante 7, il est également envisagé d'appliquer une contrainte selon un premier et un deuxième volume sur la poche filtrante 7 et notamment le compartiment de stockage 10 du deuxième composant sanguin de la poche filtrante 7.

Le procédé selon ce deuxième exemple comprend alors les étapes du procédé selon le premier exemple dans lequel la poche satellite 4 de recueil du deuxième composant sanguin est remplacée par une poche filtrante 7.

Dans ce cas, la poursuite de la compression de la poche primaire 2 est réalisée de sorte à transférer le deuxième composant sanguin dans le compartiment de stockage 10 de la poche filtrante 7.

Le procédé selon ce deuxième exemple comprend en outre l'étape de fermer le passage 11 entre le compartiment de stockage 10 et le compartiment de filtration 8.

Un procédé selon un troisième exemple est décrit ci-dessous, utilisant également une poche filtrante 7 de l'invention.

Ce procédé est adapté pour extraire au moins un premier et un deuxième composant sanguin contenus dans une poche primaire 2 d'un système à poches 1, ledit système à poches comprenant en outre au moins une poche satellite 3 de recueil du premier composant sanguin en communication fluidique avec la poche primaire 2, et une poche filtrante 7 disposée sur le chemin d'écoulement défini entre la poche primaire 2 et la poche satellite 3 de recueil du premier composant sanguin.

Comme déjà décrit, cette poche filtrante 7 comprend un compartiment de filtration 8 disposant d'un milieu filtrant et un compartiment de stockage 10 du deuxième composant sanguin, lesdits compartiments 8,10 communiquant entre eux par un passage 11 pouvant être fermé.

Le procédé selon le troisième exemple comprend les étapes de :
- compresser la poche primaire 2 de sorte à extraire le premier composant sanguin de la poche primaire 2 et à le transférer au moins en partie dans la poche satellite 3 de recueil du premier composant sanguin en passant par ladite poche filtrante 7,
- poursuivre la compression de la poche primaire 2 de sorte à extraire le deuxième composant sanguin de la poche primaire 2 et à le transférer au moins en partie dans le compartiment de stockage 10 de ladite poche filtrante 7, et
- fermer le passage 11 entre le compartiment de stockage 10 et le compartiment de filtration 8.

Avantageusement, le passage 11 entre les deux compartiments est fermé lorsque le compartiment de stockage 10 comprend une quantité prédéterminée de premier composant. Cette quantité peut être nulle, c'est-à-dire que le premier composant est transféré en totalité dans la poche satellite 3 de recueil du premier composant sanguin.

En particulier, seul le premier composant sanguin est filtré. Ainsi, lors de la compression de la poche primaire 2, le premier composant sanguin est transféré au moins en partie dans la poche satellite 3 de recueil du premier composant sanguin en passant d'abord dans le compartiment de stockage 10 de la poche filtrante 7 puis dans le compartiment de filtration 8 de ladite poche filtrante.

Dans ce troisième exemple, il est également envisagé les étapes suivantes :
- avant la compression de la poche primaire 2, contraindre le compartiment de stockage 10 de la poche filtrante 7 selon un premier volume, et
- après l'extraction du premier composant sanguin de la poche primaire, contraindre le compartiment de stockage 10 de la poche 7 filtrante selon un deuxième volume.

Avantageusement, une contrainte est également appliquée sur le compartiment de filtration 8 de sorte à filtrer sous pression le premier composant sanguin en évitant le gonflement du compartiment de filtration 8. Cette filtration sous pression réduit la perte de composant sanguin dans le compartiment de filtration 8 et améliore la qualité de la filtration.

Ainsi, le procédé selon le troisième exemple comprend avantageusement l'étape de :
- avant la compression de la poche primaire 2, contraindre le compartiment de filtration 10 de la poche filtrante selon un troisième volume.

Le troisième volume est minimal de sorte à minimiser la perte résiduelle en premier composant résultant de la filtration.

Un quatrième procédé est décrit utilisant le système à poches 1 de la figure 2 dans lequel la poche satellite 3 de recueil du premier composant sanguin et la poche satellite 4 de recueil du deuxième composant sanguin sont disposées en série. Le système comprend également en parallèle une poche satellite 5 de recueil du troisième composant sanguin.

Le procédé est notamment adapté pour extraire d'une poche primaire 2 un premier composant sanguin tel qu'un composant plasmatique riche en plasma et un deuxième composant sanguin tel qu'un composant globulaire. Le procédé comprend les étapes de :
- contraindre la poche satellite 4 de recueil du deuxième composant sanguin selon un premier volume,
- compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- poursuivre la compression de la poche primaire 2 de sorte à extraire le deuxième composant sanguin de la poche primaire et le transférer au moins en partie dans la poche satellite 5 de recueil du troisième composant sanguin.

Dans ce cas, aucun composant sanguin n'est stocké dans la poche satellite 4 de recueil du deuxième composant sanguin, le deuxième composant sanguin étant transféré dans la poche satellite 5 de recueil du troisième composant sanguin.

Ainsi, un même système à poches tel que celui représenté sur la figure 2 est utilisé au choix de l'utilisateur pour extraire deux ou trois composants sanguins contenus dans la poche primaire. Plus particulièrement, l'utilisateur peut décider au moment de la séparation de récupérer un composant leuco-plaquettaire ou non, ce qui offre une grande flexibilité par rapport aux systèmes à poches actuel dans lequel ce choix doit être fait au moment du don.

Pour mettre en œuvre ces procédés d'extraction, il est prévu un appareil pour extraire au moins un composant sanguin contenu dans une poche primaire 2.

Un appareil selon l'invention pour mettre en œuvre au moins l'un des procédés décrits ci-dessus comprend au moins un moyen de compression de la poche primaire 2 de sorte à extraire au moins un composant sanguin, particulièrement un premier et un deuxième composant sanguin et encore plus particulièrement, un premier, deuxième et troisième composant sanguin.

Selon une réalisation, le moyen de compression comprend d'une part une paroi de pressage et d'autre part une plaque de pressage entre lesquelles la poche primaire 2 est destinée à être placée. La plaque de pressage est mobile en translation pour venir comprimer la poche primaire 2.

La plaque de pressage est notamment un élément peu épais ayant une surface substantiellement plane. La plaque de pressage est rigide de sorte à pouvoir compresser la poche primaire 2, notamment contre la paroi de pressage, sans se déformer.

Le moyen de compression comprend en outre un dispositif de déplacement de ladite plaque de pressage.

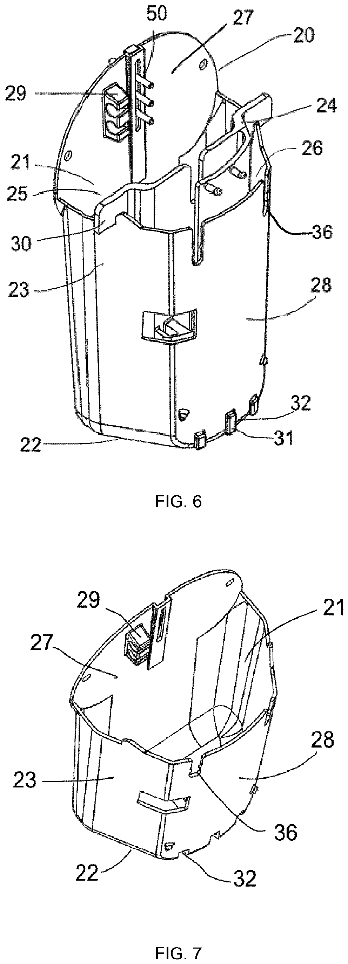

Selon une réalisation représentée sur les figures 6 à 11, la paroi de pressage et la plaque de pressage font partie d'un réceptacle 20 destiné à recevoir un système à poches 1.

Plus en détail et en relation avec les figures 6 à 11, l'appareil comprend un réceptacle 20 destiné à recevoir un système à poches 1 comprenant une poche primaire 2 en communication fluidique avec au moins une poche satellite 3,5. Ce réceptacle 20 est en particulier adapté pour être disposé dans un godet d'une centrifugeuse.

Comme illustré sur la figure 6, le réceptacle 20 comprend une cavité 21 présentant un fond 22 et une paroi latérale 23, ladite cavité 21 étant divisée par une plaque de pressage 24 en un compartiment primaire 25 destiné à recevoir la poche primaire 2 et en un compartiment secondaire 26 destiné à recevoir au moins une poche satellite 3,5 respectivement.

Sur les figures, la cavité 21 présente substantiellement une forme en demi-cylindre creux dont la base correspond au fond 22 de la cavité.

Par la suite, la partie plane de la surface latérale du demi-cylindre constitue la paroi latérale arrière 27 de la cavité 21 et la partie arrondie, la paroi latérale avant 28.

La paroi latérale arrière 27 s'étend au-delà du demi-cylindre et comprend avantageusement des logements 29 pour tubulures, comme expliqué ci-après.

La plaque de pressage 24 est notamment substantiellement perpendiculaire au fond 22 de la cavité de sorte que la poche primaire 2 et les poches satellites 3,5 sont disposées à la verticale dans le réceptacle 20.

Comme illustré sur la figure 6, le compartiment primaire 25 du réceptacle se situe à l'arrière de la cavité, à l'endroit le plus large du demi-cylindre, et le compartiment secondaire 26 à l'avant.

La paroi latérale arrière 27 de la cavité 21 constitue ainsi la paroi fixe de pressage de l'appareil contre laquelle la poche primaire 2 est compressée.

La plaque de pressage 24 est agencée pour être mobile à l'intérieur de la cavité 21 dans une première direction vers ladite paroi latérale 23 de sorte à pouvoir compresser la poche primaire 2.

La plaque de pressage 24 et la paroi latérale de la cavité 21 forment ensemble un moyen de compression de la poche primaire 2.

Lorsque la plaque de pressage 24 compresse la poche primaire 2 contre la paroi latérale 23 de la cavité, au moins une partie du contenu de la poche primaire 2 est extraite de ladite poche primaire 2 et transférée vers une poche satellite 3,4,5. Par exemple, en compressant la poche primaire 2, il est possible d'extraire un premier composant sanguin tel qu'un composant plasmatique vers une poche satellite 3 de recueil du premier composant.

En continuant à compresser la poche primaire 2, il est également possible d'extraire un deuxième composant sanguin de la poche primaire 2 et de le transférer dans une poche satellite 4 de recueil du deuxième composant sanguin.

Dans le cas où la poche primaire 2 contient un troisième composant, celui-ci peut être extrait par compression de la poche primaire 2 et transféré vers une poche satellite 5 de recueil du troisième composant sanguin.

Selon une réalisation particulière, la plaque de pressage 24 est mobile à l'intérieur de la cavité dans une deuxième direction, opposée à la première direction, vers la paroi latérale 28 de sorte à pouvoir compresser ladite au moins poche satellite 3,5.

Le déplacement de la plaque de pressage 24 dans la deuxième direction permet par exemple de chasser l'air contenu dans une poche satellite 3 contenant un composant sanguin tel qu'un composant plasmatique et/ou de transférer une solution additive contenue dans une poche satellite 5 vers la poche primaire 2 contenant un composant sanguin séparé tel qu'un composant globulaire.

Pour pouvoir déplacer la plaque de pressage 24, celle-ci est pourvue de saillies dépassant du réceptacle 20 et sur lesquelles une pression est exercée afin de déplacer la plaque de pressage 24 à l'intérieur de la cavité du réceptacle 20 dans un sens ou dans l'autre.

Sur les figures, les saillies sont sous forme d'ailettes 30 et d'ergots 31. La plaque de pressage 24 comporte une paire d'ailettes 30 disposée sur le haut de la plaque de pressage 24 et trois ergots 31 disposés sur le bas de la plaque de pressage 24.

Les ergots 31 sont agencés pour s'engager dans des ouvertures 32 pratiquées dans la paroi latérale 23 de la cavité 21.

En variante, la plaque de pressage 24 est amovible, c'est-à-dire qu'elle peut être retirée du réceptacle. Dans ce cas, la mise en place du système à poches dans la cavité est simplifiée. Il suffit de positionner la poche primaire 2 et/ou les poches satellites 3,4,5 de part et d'autre de ladite plaque de pressage 24 puis de glisser l'ensemble de la plaque de pressage 24 et des poches 2,3,4,5 dans la cavité 21 du réceptacle 20.

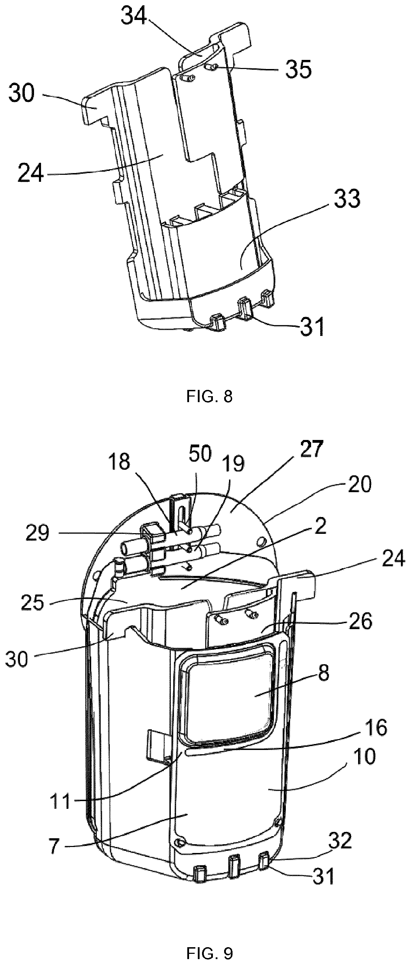

En relation avec la figure 8, pour faciliter le positionnement des poches satellites 3,4,5, la plaque de pressage 24 comprend une bordure formant une rainure 33 sur un ou plusieurs bords de la plaque de pressage 24, dans lesquelles la ou les poches satellites 3,4,5 peuvent être glissées.

Avantageusement, la géométrie de la surface de la plaque de pressage 24 en contact avec la poche primaire 2 correspond sensiblement à la géométrie de la poche primaire 2 remplie. Ainsi, la pression exercée sur la poche primaire 2 lors du déplacement de la plaque de pressage 24 est substantiellement constante sur toute la surface de la poche primaire 2, ce qui permet une meilleure extraction des composants sanguins.

Sur la figure 8, la surface arrière de la plaque de pressage 24 est substantiellement concave.

La géométrie de la surface de la plaque de pressage 24 en contact avec ladite au moins une poche satellite 3,5 correspond sensiblement à la géométrie de la paroi latérale de la cavité 21 vers laquelle la plaque de pressage 24 est déplacée pour comprimer ladite poche satellite 3,5. Cette correspondance de géométrie permet notamment de vidanger complètement la poche satellite 5 contenant une solution additive.

Sur la figure 8, la surface avant de la plaque de pressage 24 présente substantiellement une surface arrondie dont la géométrie correspond à la géométrie de la surface intérieure de paroi latérale 23 avant de la cavité 21.

Lorsque le système à poches 1 comprend une unité de filtration 6, il est avantageux de prévoir que le réceptacle 20 et notamment la plaque de pressage 24 comprenne un logement 34 pour une telle unité de filtration 6.

Ainsi, l'unité de filtration 6 reste en place durant la centrifugation, ce qui permet d'assurer son intégrité et/ou l'intégrité des poches du système à poches 1.

En relation avec la figure 8, le logement 34 pour l'unité de filtration 6 est arrangé sur la plaque de pressage 24 et comprend avantageusement une ouverture en haut et en bas du logement afin de faciliter le passage des tubulures.

Aussi, afin de maintenir les poches en position verticale dans le réceptacle 20, il est utile de pourvoir ledit réceptacle 20 d'une ou plusieurs tiges 35 destinées à recevoir des œillets pourvus sur la poche primaire 2 et/ou satellites 3,4,5.

Sur la figure 8, deux tiges 35 sont arrangées sur la surface avant de la plaque de pressage 24 et notamment sur la surface avant du logement 34 pour l'unité de filtration 6. Ces tiges 35 maintiennent en position verticale au moins une poche satellite 3,5.

Selon une réalisation particulière, la paroi latérale 23 de la cavité 21 est pourvue sur sa surface extérieure d'un moyen d'association d'une poche satellite, notamment de la poche satellite 4 de recueil d'un deuxième composant sanguin.

Par exemple, ce moyen d'association est une paire d'ouvertures 36 pratiquée dans la paroi latérale avant 28 de la cavité 21, dans laquelle les orifices 12,13 d'entrée et sortie d'une poche satellite 4,7 se glissent. Pour cela, chacun des orifices 12,13 d'entrée et sortie de ladite poche satellite se présente sous forme d'une pièce injectée soudée sur la surface latérale de ladite poche.

La configuration particulière de ce réceptacle 20 avec notamment sa plaque de pressage mobile 24 permet le transfert des composants sanguins séparés sans retirer aucune poche du réceptacle 20 ce qui est avantageux pour au moins deux raisons.

D'abord, le système à poches 1 dans son ensemble, et plus particulièrement la poche primaire 2, n'est pas retiré du réceptacle 20, ce qui limite le risque de mélanger à nouveau les composants sanguins séparés par centrifugation. Ensuite, aucune manipulation du système à poches 1 n'est nécessaire, ce qui est un gain de temps pour l'opérateur qui n'a qu'à positionner le réceptacle 20 sur un appareil dédié afin de réaliser l'extraction des composants dans les différentes poches satellites.

Dans une réalisation non représentée, le réceptacle 20 est adossé ou intégré à un autre réceptacle 20, de sorte à former un réceptacle jumelé comprenant deux cavités 21 destinées à recevoir chacune un système à poches 1.

De façon avantageuse, le réceptacle 20, dans sa version simple ou jumelée, est adapté pour être disposé dans un godet d'une centrifugeuse. La place disponible dans le réceptacle 20 pour recevoir le système à poche est optimisée grâce à la plaque de pressage mobile 24 qui non seulement divise la cavité 21 du réceptacle 20 en deux compartiments 25,26 mais aussi permet la compression des poches du système à poches.

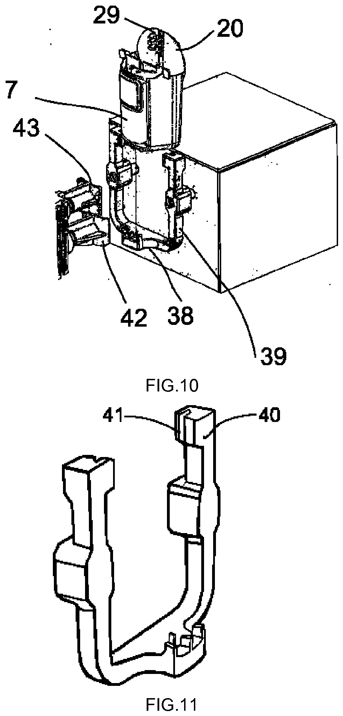

En relation avec les figures 10 et 11 et selon un exemple particulier, un appareil est décrit pour extraire au moins un composant sanguin contenu dans une poche primaire 2 d'un système à poches 1, ledit système à poches 1 comprenant en outre au moins une poche satellite 3,5 de recueil dudit composant sanguin.

L'appareil comprend :
- un réceptacle 20 tel que décrit ci-dessus,
- un moyen de réception 38 dudit réceptacle 20, et
- un dispositif de déplacement 39 de la plaque de pressage 24 dans ledit réceptacle 20.

Le dispositif de déplacement 39 de la plaque de pressage 24 comprend notamment un moyen d'engagement de la plaque de pressage et un actionneur en translation dudit moyen d'engagement présentant par exemple un moteur en liaison avec une vis sans fin.

Le moyen d'engagement comprend un support 40 muni de fentes 41 dans lesquelles les saillies de la plaque de pressage 24 viennent s'engager.

Le dispositif de déplacement 39 de la plaque de pressage 24 est agencé pour permettre le déplacement de la plaque de pressage 24 à l'intérieur de la cavité 21 du réceptacle 20 dans une première direction. Avantageusement, le déplacement de la plaque de pressage 24 est possible dans une première direction et dans une deuxième direction opposée à la première direction.

Comme déjà décrit ci-dessus, dans le cas d'un système à poches 1 tel que représenté en figure 2 et 3, dans lequel la poche satellite 3 de recueil d'un premier composant sanguin et la poche satellite 4 de recueil d'un deuxième composant sanguin ou la poche filtrante 7, sont disposées en série, il est envisagé de disposer ladite poche de recueil 4 du deuxième composant sanguin ou la poche filtrante 7 sur la surface extérieure de la paroi latérale 23 de la cavité 21 du réceptacle 20, comme représenté sur la figure 9.

Dans ce cas, pour permettre de transférer le premier composant sanguin dans la poche satellite 3 de recueil du premier composant sanguin en passant par la poche satellite 4 de recueil du deuxième composant sanguin ou la poche filtrante 7, un procédé particulier consiste à contraindre la poche satellite 4 de recueil du deuxième composant sanguin ou le compartiment de stockage 10 de la poche filtrante 7 selon un premier volume, suffisamment faible pour permettre un transfert rapide et complet du premier composant sanguin dans sa poche satellite 3 dédiée.

Aussi, après l'extraction du premier composant sanguin de la poche primaire 2, le procédé consiste à contraindre la poche satellite 4 de recueil du deuxième composant sanguin ou le compartiment de stockage 10 de la poche filtrante 7 selon un deuxième volume, qui correspond généralement au volume désiré de deuxième composant sanguin.

Pour cela, l'appareil comprend en outre un moyen de contrainte de la poche de recueil 4 du deuxième composant sanguin ou du compartiment de stockage 10 de la poche filtrante 7 selon un premier et un deuxième volume.

Le moyen de contrainte comprend une paroi fixe de contrainte et une plaque de contrainte entre lesquelles la poche satellite 4 de recueil du deuxième composant sanguin est destinée à être placée. La plaque de contrainte est mobile en translation pour venir contraindre ladite poche satellite.

Le moyen de contrainte comprend en outre un dispositif de déplacement de la plaque de contrainte.

Afin de fixer ou déterminer le premier et le deuxième volume de contrainte, il est avantageux de prévoir en outre un capteur de pression sur la plaque de contrainte.

En particulier, lorsque l'appareil comprend un réceptacle 20 tel que décrit ci-dessus, la paroi fixe de contrainte correspond à la paroi latérale avant 28 de la cavité 21 du réceptacle 20.

L'appareil comprend alors une plaque de contrainte 42 mobile pour contraindre une poche satellite disposée sur la surface extérieure du réceptacle 20 et un dispositif de déplacement de ladite plaque de contrainte. En particulier, la plaque de contrainte 42 est agencée pour contraindre une poche satellite, notamment une poche satellite 4 de recueil du deuxième composant sanguin selon un premier et un deuxième volume.

Le dispositif de déplacement de la plaque de contrainte 42 comprend notamment un moyen d'engagement de la plaque de contrainte et un actionneur en translation dudit moyen d'engagement présentant par exemple un moteur en liaison avec une vis sans fin.

La détermination ou la fixation du volume de composant sanguin dans la poche de recueil 4 ou le compartiment de stockage 10 du deuxième composant sanguin dépend non seulement de la distance entre la paroi de contrainte et la plaque de contrainte, mais aussi de la pression détectée sur ladite plaque de contrainte.

Dans une autre réalisation, dans le cas où le système à poches 1 destiné à être disposé dans un réceptacle 20 comprend une poche filtrante 7 comme décrit ci-dessus et illustré sur la figure 3, la plaque de contrainte 42 est agencée pour contraindre le compartiment de stockage 10 du deuxième composant sanguin de la poche filtrante 7.

En autre variante représentée sur la figure 10, une autre plaque de contrainte 43 est agencée pour contraindre le compartiment de filtration 8 de la poche filtrante 7 pour réaliser une filtration sous pression.

En variante non représentée, la plaque de contrainte 42 est agencée pour contraindre non seulement le compartiment de stockage 10 de la poche filtrante 7 mais aussi le compartiment de filtration 8.

L'appareil comprend en outre un dispositif de contrôle destiné à contrôler l'écoulement des fluides dans le système à poches 1.

En particulier, le dispositif de contrôle comprend un ensemble de clamps pour les tubulures associant entre elles la poche primaire 2 et les poches satellites 3,4,5.

Dans le cas où le système à poches 1 destiné à être disposé dans le réceptacle 20 comprend une poche filtrante 7 comme décrit ci-dessus et illustré sur la figure 3, le dispositif de contrôle comprend en outre au moins un clamp 46 pour fermer le passage 11 entre le compartiment de stockage 10 et le compartiment de filtration 8 de la poche filtrante 7.

Par exemple, le dispositif de contrôle comprend entre un et six clamps. Chaque clamp est apte à pincer une tubulure du système à poches 1 pour bloquer l'écoulement fluidique à l'intérieur de ladite tubulure.

Dans un exemple particulier, les clamps comprennent une soudeuse apte à souder la tubulure de sorte à fermer définitivement l'écoulement dans ladite tubulure, après extraction et transfert des composants sanguins séparés dans les poches appropriées.

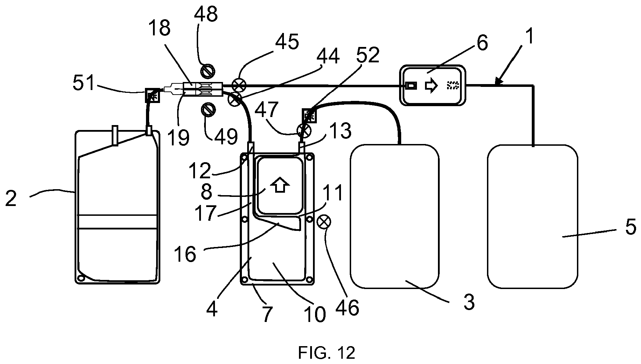

Selon la figure 12, lorsqu'on dispose le système à poches dans l'appareil, la tubulure reliant la poche primaire 2 et la poche filtrante 7 est positionnée dans un premier clamp 44 ; la tubulure reliant la poche primaire 2 et la poche satellite 5 de recueil du troisième composant sanguin est positionnée dans un deuxième clamp 45 ; le passage 11 entre le compartiment de stockage 10 et le compartiment de filtration 8 de la poche filtrante 7 est positionné dans un troisième clamp 46 ; la tubulure reliant la poche filtrante 7 et la poche satellite 3 de recueil du premier composant sanguin est positionnée dans un quatrième clamp 47.

Chaque clamp est en outre muni d'un détecteur optique afin de signaler le bon positionnement de la tubulure dans le clamp.

Selon une réalisation, le dispositif de contrôle comprend un ensemble de dispositifs de rupture 48,49 d'au moins un élément de fermeture 18,19 comportant une zone de fragilité, ledit élément de fermeture étant disposé à l'intérieur de l'une des tubulures associant entre elles la poche primaire 2 et une poche satellite 3,4,5. Un tel dispositif de rupture est décrit dans le document FR 2 968 568.

En particulier, chacun desdits dispositifs de rupture comporte un ensemble de réception d'au moins une tubulure, ledit ensemble comprenant un élément fixe pourvu d'un premier logement 29 destiné à recevoir une première portion de ladite tubulure, un élément mobile 50 pourvu d'un deuxième logement destiné à recevoir une deuxième portion de ladite tubulure souple, et un organe d'entraînement en déplacement de l'élément mobile, de sorte à pouvoir provoquer la rupture de la zone de fragilité de l'élément de fermeture lorsque la tubulure est placée dans l'ensemble de réception.

Sur la figure 6, le logement 29 de l'élément fixe du dispositif de rupture est arrangé sur la paroi latérale arrière 27 de la cavité 21 du réceptacle 20. L'élément mobile 50 est sous forme de tiges qui traversent une ouverture pratiquée dans la paroi latérale de la cavité pour venir recevoir la deuxième portion de la tubulure.

L'appareil comprend en outre un dispositif de détection afin de détecter l'interface entre les différents composants sanguins et/ou le passage d'un composant dans les tubulures du système à poches 1.

En particulier, le dispositif de détection comprend un ensemble de détecteurs optiques placés au niveau de la poche primaire 2 et/ou des tubulures du système à poches 1 afin de détecter l'interface entre les différents composants sanguins et/ou le passage d'un composant dans les tubulures.

Les détecteurs optiques sont arrangés sur le réceptacle 20 ou directement sur l'appareil.

Selon la figure 12, une fois le système à poches disposé dans l'appareil, un premier détecteur 51 est positionné à proximité de la sortie de la poche primaire 2, sur la tubulure reliant la poche primaire 2 aux poches satellite 3,4,5. Ce détecteur détecte l'interface entre les différents composants sanguins contenus dans la poche primaire 2, mais aussi l'interface entre de l'air et un composant sanguin.

Un deuxième détecteur 52 est positionné à la sortie de la poche satellite 4 de recueil du deuxième composant sanguin ou de la poche filtrante 7 et permet de détecter l'interface entre l'air et le composant plasmatique.

Dans une réalisation particulière, le dispositif de détection comprend un ou plusieurs détecteurs de pression agencés sur la plaque de contrainte afin de déterminer la pression exercée par la poche satellite de recueil du deuxième composant sanguin. L'atteinte d'une pression déterminée traduit l'atteinte du volume attendu de deuxième composant sanguin.

L'appareil comprend également un système de pilotage apte à commander les dispositifs de déplacement et de contrôle, de sorte à transférer les composants sanguins de la poche primaire 2 dans les poches satellites 3,4,5.

Le système de pilotage comprend notamment un microprocesseur conçu pour exécuter un programme de commandes. L'exécution de ce programme permet au système de pilotage de piloter les dispositifs de déplacement et de contrôle en fonction par exemple des signaux reçus par les détecteurs optiques et/ou les détecteurs de pression.

Le système de pilotage permet de mettre en œuvre un procédé de traitement d'un fluide biologique comme décrit ci-dessus.

Enfin, il est décrit un procédé pour extraire au moins un premier composant sanguin contenu dans une poche primaire 2 d'un système à poches 1, ladite poche primaire étant en communication fluidique avec au moins une poche satellite 4 de recueil du premier composant sanguin, ledit procédé étant réalisé à l'aide d'un appareil tel que décrit ci-dessus. Le procédé comprend les étapes de :
- disposer un système à poches 1 comprenant une poche primaire 2 contenant au moins un premier composant sanguin séparé dans le réceptacle 20 de l'appareil,
- disposer ledit réceptacle 20 contenant le système à poches 1 sur le moyen de réception 38 dudit appareil,
- mettre en fonction l'appareil pour extraire ledit au moins un composant sanguin de la poche primaire 2.

Ainsi, il est possible de réaliser l'extraction des différents composants sanguins d'un système à poches 1, sans manipulation de celui-ci une fois placé dans le réceptacle 20 au début du processus. Aucune manipulation du système à poches 1 n'est nécessaire, ce qui est un gain de temps pour l'opérateur qui n'a qu'à positionner le réceptacle 20 sur un appareil dédié afin de réaliser l'extraction des composants dans les différentes poches satellites 3,4,5.

Notamment, la mise en fonction de l'appareil comprend, via le système de pilotage, l'action de déplacer la plaque de pressage 24 du réceptacle 20 pour compresser la poche primaire 2 du système à poches 1 de sorte à extraire le premier composant de la poche primaire 2 et à le transférer dans la poche satellite 3.

Dans le cas d'un système à poches 1 comprenant une poche satellite de recueil 4 d'un deuxième composant sanguin disposée en série (figure 2), et dans le cas d'un appareil comprenant une plaque de contrainte mobile 42 pour contraindre cette poche satellite disposée sur la surface extérieure du réceptacle, la mise en fonction de l'appareil comprend les actions de :
- déplacer la plaque de contrainte 42 pour contraindre la poche satellite 4 de recueil du deuxième composant sanguin selon un premier volume,
- déplacer la plaque de pressage 24 pour compresser la poche primaire 2 de sorte à extraire le premier composant sanguin de la poche primaire 2 et le transférer au moins en partie dans la poche satellite 3 de recueil du premier composant sanguin en passant par la poche satellite 4 de recueil du deuxième composant sanguin contrainte selon le premier volume,
- après l'extraction du premier composant sanguin, déplacer la plaque de contrainte 42 pour contraindre la poche satellite 4 de recueil du deuxième composant sanguin selon un deuxième volume,
- déplacer la plaque de pressage 24 pour poursuivre la compression de la poche primaire 2 de sorte à extraire le deuxième composant sanguin de la poche primaire et le transférer au moins en partie dans la poche satellite 4 de recueil du deuxième composant sanguin contrainte selon le deuxième volume.

En relation avec un système à poches 1 comprenant une poche filtrante 7, la mise en fonction de l'appareil comprend les actions de :
- déplacer la plaque de pressage 24 pour compresser la poche primaire 2 de sorte à extraire le premier composant sanguin de la poche primaire 2 et à le transférer au moins en partie dans la poche satellite 3 de recueil du premier composant sanguin en passant par ladite poche filtrante 7,
- poursuivre le déplacement de la plaque de pressage 24 pour poursuivre la compression de la poche primaire 2 de sorte à extraire le deuxième composant sanguin de la poche primaire 2 et à le transférer au moins en partie dans le compartiment de stockage 10 de ladite poche filtrante 7, et
- actionner le dispositif de contrôle pour fermer le passage 11 entre le compartiment de stockage 10 et le compartiment de filtration 8.

Selon un exemple particulier, un système à poches 1 tel que celui de la figure 3 est disposé dans un réceptacle 20 comme montré partiellement sur la figure 9.

En relation avec la figure 12, le système de pilotage exécute les actions suivantes :
- fermer les clamps 44,45 à la sortie de la poche primaire 2,
- rompre les éléments de fermeture 18,19 à la sortie de la poche primaire 2
- ouvrir le clamp 45 pour permettre la communication fluidique entre la poche primaire 2 et la poche satellite de recueil du troisième composant sanguin 5,
- déplacer la plaque de pressage du réceptacle selon une première direction de sorte à compresser la poche primaire 2 et à chasser l'air de la poche primaire vers l'unité de filtration 6,
- à la détection par le détecteur 51 de l'interface air/plasma, fermer le clamp 45,
- ouvrir le clamp 44 pour permettre l'écoulement fluidique entre la poche primaire 2 et la poche filtrante 4,
- déplacer la plaque de contrainte 42 selon la première direction de sorte à contraindre le compartiment de stockage 10 du deuxième composant sanguin selon un premier volume,
- déplacer la plaque de pressage 24 du réceptacle 20 selon la première direction de sorte à compresser la poche primaire 2 et à transférer le premier composant sanguin dans la poche satellite 3 de recueil du premier composant sanguin en passant par le compartiment de stockage 10 et le compartiment de filtration 8 de la poche de stockage 7,
- à la détection par le détecteur 51 du deuxième composant sanguin, fermer le clamp 46 pour fermer le passage 11 entre le compartiment de stockage 10 de la poche filtrante 7 et le compartiment de filtration 8,
- déplacer la plaque de contrainte 42 selon la première direction de sorte à contraindre le compartiment de stockage 10 de la poche filtrante 7 selon un deuxième volume,
- déplacer la plaque de pressage 24 du réceptacle 20 selon la première direction de sorte à compresser la poche primaire 2 et à transférer le deuxième composant sanguin dans le compartiment de stockage 10 du deuxième composant sanguin,
- à la détection par le détecteur de pression placé sur la plaque de contrainte 42 d'une certaine valeur de pression correspondant à un volume déterminé de deuxième composant sanguin, fermer le clamp 44 pour interrompre la communication entre la poche primaire 2 et la poche filtrante 7,
- déplacer la plaque de pressage 24 dans une deuxième direction opposée à la première direction de sorte à compresser la poche satellite 3 de recueil du premier composant sanguin et à chasser l'air de ladite poche satellite,
- à la détection par le détecteur 52 de l'interface air/plasma, fermer le clamp 47 pour interrompre la communication entre la poche filtrante 7 et la poche satellite 3 de recueil du premier composant,
- ouvrir le clamp 45 pour permettre la communication entre la poche primaire 1 et la poche satellite 5 de recueil du troisième composant sanguin,
- déplacer la plaque de pressage 24 dans la deuxième direction de sorte à compresser la poche satellite 5 contenant la solution additive et à transférer ladite solution additive dans la poche primaire 2.

Une fois ces commandes exécutées, l'opérateur retire le système à poches 1 du réceptacle 20. Dans un exemple particulier, le composant globulaire et la solution additive contenus dans la poche primaire 2 sont ensuite filtrés par gravité en suspendant la poche primaire 2 à un portique.

## Revendications

1. Procédé pour extraire au moins un premier et un deuxième composant sanguin contenus dans une poche primaire (2) d'un système à poches (1), ledit système à poches (1) comprenant en outre au moins une poche satellite (3) de recueil du premier composant sanguin en communication fluidique avec la poche primaire (2), et une poche filtrante (7) disposée sur le chemin d'écoulement défini entre la poche primaire (2) et la poche satellite (3) de recueil du premier composant sanguin, la poche filtrante (7) comprenant un compartiment de filtration (8) disposant d'un milieu filtrant et un compartiment de stockage (10) du deuxième composant sanguin, lesdits compartiments (8,10) communiquant entre eux par un passage (11) pouvant être fermé, ledit procédé comprenant les étapes de :
- compresser la poche primaire (2) de sorte à extraire le premier composant sanguin de la poche primaire (2) et à le transférer au moins en partie dans la poche satellite (3) de recueil du premier composant sanguin en passant par ladite poche filtrante (7),
- poursuivre la compression de la poche primaire (2) de sorte à extraire le deuxième composant sanguin de la poche primaire (2) et à le transférer au moins en partie dans le compartiment de stockage (10) de ladite poche filtrante (7),
**caractérisé en ce que** ledit procédé comprend l'étape de
- fermer le passage (11) entre le compartiment de stockage (10) et le compartiment de filtration (8).

2. Procédé selon la revendication 1, **caractérisé en ce que** l'étape de fermeture du passage (11) est réalisée lorsque le compartiment de stockage (10) de la poche filtrante (7) comprend une quantité prédéterminée de premier composant.

3. Procédé selon la revendication 1 ou 2, **caractérisé en ce que**, lors de la compression de la poche primaire (2), le premier composant sanguin est transféré au moins en partie dans la poche satellite (3) de recueil du premier composant sanguin en passant d'abord dans le compartiment de stockage (10) de la poche filtrante (7) puis dans le compartiment de filtration (8) de la poche filtrante (7).

4. Procédé selon l'une quelconque des revendications 1 à 3, **caractérisé en ce que** ladite poche filtrante (7) est pourvue d'un orifice (12) d'entrée en communication avec le compartiment de stockage (10) et un orifice de sortie (13) en communication avec le compartiment de filtration (8).

5. Procédé selon la revendication 4, **caractérisé en ce que** les orifices (12,13) d'entrée et de sortie de la poche filtrante (7) sont disposés au voisinage d'un bord supérieur de la poche filtrante (7).

6. Procédé selon l'une quelconque des revendications 1 à 5, **caractérisé en ce que** la poche filtrante (7) comprend une première et deuxième feuille (14,15) en matière thermoplastique souple solidarisées l'une à l'autre sur leur périphérie de sorte à former un volume intérieur, ladite poche (7) comportant un cordon de soudure (16) entre les feuilles (14,15) agencé pour former le compartiment de filtration (8) et le compartiment de stockage (10), ledit cordon de soudure (16) étant agencé pour former ledit passage (11) entre les compartiments.

7. Procédé selon la revendication 6, **caractérisé en ce que** le passage (11) entre le compartiment de filtration (8) et le compartiment de stockage (10) est fermé par soudage des deux feuilles (14,15).

8. Procédé selon la revendication 6 ou 7, **caractérisé en ce que** le cordon de soudure (16) comprend une partie fragile de sorte à pouvoir séparer le compartiment de filtration (8) et le compartiment de stockage (10).

9. Procédé selon l'une quelconque des revendications 1 à 8, **caractérisé en ce qu'**il comprend en outre les étapes de :
- avant la compression de la poche primaire (2), contraindre le compartiment de stockage (10) de la poche filtrante (7) selon un premier volume, et
- après l'extraction du premier composant sanguin de la poche primaire (2), contraindre le compartiment de stockage (10) de la poche filtrante (7) selon un deuxième volume.

10. Procédé selon l'une quelconque des revendications 1 à 9, **caractérisé en ce qu'**il comprend en outre l'étape de :
- avant la compression de la poche primaire (2), contraindre le compartiment de filtration (8) de la poche filtrante (7) selon un troisième volume.

11. Poche filtrante pour la mise en œuvre du procédé selon l'une quelconque des revendications 1 à 10, ladite poche filtrante (7) comprenant une première et deuxième feuille (14,15) en matière thermoplastique souples solidarisées l'une à l'autre sur leur périphérie de sorte à former un volume intérieur, au moins un orifice d'entrée (12) et un orifice de sortie (13), et un cordon de soudure (16) entre les feuilles (14,15) agencé pour former le compartiment de filtration (8) et le compartiment de stockage (10), **caractérisé en ce que** ledit cordon de soudure (16) comprend une partie horizontale et une partie verticale agencée pour former avec un bord de la poche filtrante (7) un canal (17) en communication fluidique avec l'un des orifices d'entrée ou sortie (12,13) de la poche filtrante (7).

12. Appareil pour mettre en œuvre le procédé selon l'une quelconque des revendications 1 à 10, **caractérisé en ce qu'**il comprend :
- un moyen de compression de la poche primaire (2) de sorte à extraire au moins un composant sanguin, et
- un dispositif de contrôle destiné à contrôler l'écoulement des fluides dans le système à poches (1), ledit dispositif de contrôle comprenant au moins un clamp (46) pour fermer le passage (11) entre le compartiment de stockage (10) et le compartiment de filtration (8).

13. Appareil selon la revendication 12, **caractérisé en ce qu'**il comprend un réceptacle (20) destiné à recevoir un système à poches (1), ledit réceptacle comprenant une cavité (21) présentant un fond (22) et une paroi latérale (23), ladite cavité (21) étant divisée par une plaque de pressage (24) perpendiculaire au fond (22) de la cavité (21), en un compartiment primaire (25) destiné à recevoir la poche primaire (2) et en un compartiment secondaire (26) destiné à recevoir au moins une poche satellite (3,5) respectivement, ladite plaque de pressage (24) étant mobile à l'intérieur de ladite cavité (21) dans une première direction vers ladite paroi latérale (23) de sorte à compresser la poche primaire (2).

## Patentansprüche

1. Verfahren zur Extraktion von mindestens einem ersten und einem zweiten Blutbestandteil, die in einer Primärtasche (2) eines Systems mit Taschen (1) enthalten sind, wobei das System mit Taschen (1) weiter mindestens eine Satellitentasche (3) zur Aufnahme des ersten Blutbestandteils in Fluidverbindung mit der Primärtasche (2), und eine Filtertasche (7) umfasst, die am Strömungspfad angeordnet ist, der zwischen der Primärtasche (2) und der Satellitentasche (3) zur Aufnahme des ersten Blutbestandteils definiert ist, wobei die Filtertasche (7) ein Filterungsfach (8) umfasst, das über ein Filtermedium verfügt und ein Speicherfach (10) des zweiten Blutbestandteils, wobei die Fächer (8, 10) durch einen Durchlass (11) miteinander verbunden sind, der geschlossen werden kann, wobei das Verfahren die folgenden Schritte umfasst:
- Komprimieren der Primärtasche (2) derart, um den ersten Blutbestandteil aus der Primärtasche (2) zu extrahieren und ihn mindestens teilweise in die Satellitentasche (3) zur Aufnahme des ersten Blutbestandteils beim Durchlaufen durch die Filtertasche (7) zu übertragen,
- Fortsetzten des Komprimierens der Primärtasche (2) derart, um den zweiten Blutbestandteil aus der Primärtasche (2) zu extrahieren und ihn mindestens teilweise in das Speicherfach (10) der Filtertasche (7) zu übertragen,
**dadurch gekennzeichnet, dass** das Verfahren den folgenden Schritt umfasst
- Schließen des Durchlasses (11) zwischen dem Speicherfach (10) und dem Filterungsfach (8).

2. Verfahren nach Anspruch 1, **dadurch gekennzeichnet, dass** der Schritt des Schließens des Durchlasses (11) ausgeführt wird, wenn das Speicherfach (10) der Filtertasche (7) eine vorbestimmte Menge eines ersten Bestandteils umfasst.

3. Verfahren nach Anspruch 1 oder 2, **dadurch gekennzeichnet, dass** beim Komprimieren der Primärtasche (2) der erste Blutbestandteil mindestens teilweise in die Satellitentasche (3) zur Aufnahme des ersten Blutbestandteils beim Durchlaufen zuerst durch das Speicherfach (10) der Filtertasche (7), und danach durch das Filterungsfach (8) der Filtertasche (7) übertragen wird.

4. Verfahren nach einem der Ansprüche 1 bis 3, **dadurch gekennzeichnet, dass** die Filtertasche (7) mit einer Eingangsöffnung (12) in Verbindung mit dem Speicherfach (10) und einer Ausgangsöffnung (13) in Verbindung mit dem Filterungsfach (8) versehen ist.

5. Verfahren nach Anspruch 4, **dadurch gekennzeichnet, dass** die Eingangs- und Ausgangsöffnungen (12, 13) der Filtertasche (7) in der Nähe eines oberen Randes der Filtertasche (7) angeordnet sind.

6. Verfahren nach einem der Ansprüche 1 bis 5, **dadurch gekennzeichnet, dass** die Filtertasche (7) eine erste und zweite Folie (14, 15) aus biegsamem thermoplastischem Material, die an ihrer Peripherie derart fest miteinander verbunden sind, um ein Innenvolumen zu bilden, wobei die Tasche (7) eine Schweißraupe (16) zwischen den Folien (14, 15) beinhaltet, die angeordnet ist, um das Filterungsfach (8) und das Speicherfach (10) zu bilden, wobei die Schweißraupe (16) angeordnet ist, um den Durchlass (11) zwischen den Fächern zu bilden.

7. Verfahren nach Anspruch 6, **dadurch gekennzeichnet, dass** der Durchlass (11) zwischen dem Filterungsfach (8) und dem Speicherfach (10) durch Schweißen der beiden Folien (14, 15) geschlossen wird.

8. Verfahren nach Anspruch 6 oder 7, **dadurch gekennzeichnet, dass** die Schweißraupe (16) einen fragilen Teil derart umfasst, um das Filterungsfach (8) und das Speicherfach (10) trennen zu können.

9. Verfahren nach einem der Ansprüche 1 bis 8, **dadurch gekennzeichnet, dass** es weiter die folgenden Schritte umfasst:
- vor dem Komprimieren der Primärtasche (2), das Beaufschlagen des Speicherfaches (10) der Filtertasche (7) entsprechend einem ersten Volumen, und
- nach dem Extrahieren des ersten Blutbestandteils aus der Primärtasche (2), das Beaufschlagen des Speicherfaches (10) der Filtertasche (7) entsprechend einem zweiten Volumen.

10. Verfahren nach einem der Ansprüche 1 bis 9, **dadurch gekennzeichnet, dass** es weiter den folgenden Schritt umfasst:
- vor dem Komprimieren der Primärtasche (2), das Beaufschlagen des Filterungsfaches (8) der Filtertasche (7) entsprechend einem dritten Volumen.

11. Filtertasche zur Anwendung des Verfahrens nach einem der Ansprüche 1 bis 10, wobei die Filtertasche (7) eine erste und zweite Folie (14, 15) aus biegsamem thermoplastischem Material, die an ihrer Peripherie derart fest miteinander verbunden sind, um ein Innenvolumen, mindestens eine Eingangsöffnung (12) und eine Ausgangsöffnung (13) und eine Schweißraupe (16) zwischen den Folien (14, 15) zu bilden, die angeordnet ist, um das Filterungsfach (8) und das Speicherfach (10) zu bilden, **dadurch gekennzeichnet, dass** die Schweißraupe (16) einen horizontalen Teil und einen vertikalen Teil umfasst, der angeordnet ist, um mit einem Rand der Filtertasche (7) einen Kanal (17) in Fluidverbindung mit einer der Eingangs-oder Ausgangsöffnungen (12, 13) der Filtertasche (7) zu bilden.

12. Einrichtung zur Anwendung des Verfahrens nach einem der Ansprüche 1 bis 10, **dadurch gekennzeichnet, dass** sie umfasst:
- ein Mittel zum Komprimieren der Primärtasche (2) derart, um mindestens einen Blutbestandteil zu extrahieren, und
- eine Kontrollvorrichtung, die dazu bestimmt ist, die Strömung der Fluide in dem System mit Taschen (1) zu kontrollieren, wobei die Kontrollvorrichtung mindestens eine Klemme (46) umfasst, um den Durchlass (11) zwischen dem Speicherfach (10) und dem Filterungsfach (8) zu schließen.

13. Einrichtung nach Anspruch 12, **dadurch gekennzeichnet, dass** sie einen Behälter (20) umfasst, der dazu bestimmt ist, ein System mit Taschen (1) aufzunehmen, wobei der Behälter einen Hohlraum (21) umfasst, der einen Boden (22) und eine Seitenwand (23) aufweist, wobei der Hohlraum (21) durch eine zum Boden (22) des Hohlraumes (21) senkrechte Pressplatte (24) in ein Primärfach (25), das dazu bestimmt ist, die Primärtasche (2) aufzunehmen, und in ein Sekundärfach (26), das dazu bestimmt ist, jeweils mindestens eine Satellitentasche (3, 5) aufzunehmen geteilt wird, wobei die Pressplatte (24) im Inneren des Hohlraumes (21) in einer ersten Richtung derart zur Seitenwand (23) hin beweglich ist, um die Primärtasche (2) zu komprimieren.

## Claims

1. Method for extracting at least a first and a second blood component contained in a first bag (2) of a bag system (1), said bag system (1) further comprising at least one satellite bag (3) for collecting the first blood component in fluidic communication with the first bag (2), and a filter bag (7) arranged in the flow path defined between the first bag (2) and the satellite bag (3) for collecting the first blood component, the filter bag (7) comprising a filtration compartment (8) having a filtering medium and a storage compartment (10) for the second blood component, said compartments (8, 10) communicating with each other via a passage (11) that can be closed, said method comprising the steps of:
- compressing the first bag (2) so as to extract the first blood component from the first bag (2) and transfer it at least partly into the satellite bag (3) for collecting the first blood component passing via said filter bag (7),
- further compressing the first bag (2) so as to extract the second blood component from the first bag (2) and to transfer it at least partly into the storage compartment (10) of said filter bag (7),
**characterised in that** said method comprises the step of
- closing the passage (11) between the storage compartment (10) and the filtration compartment (8).

2. Method according to claim 1, **characterised in that** the step of closing the passage (11) is performed when the storage compartment (10) of the filter bag (7) comprises a predetermined amount of the first component.

3. Method according to claim 1 or 2, **characterised in that** upon the compression of the first bag (2), the first blood component is transferred at least partly into the satellite bag (3) for collecting the first blood component by passing firstly into the storage compartment (10) of the filter bag (7) then into the filtration compartment (8) of the filter bag (7).

4. Method according to any of claims 1 to 3, **characterised in that** said filter bag (7) is provided with an inlet opening (12) in communication with the storage compartment (10) and an outlet opening (13) in communication with the filtration compartment (8).

5. Method according to claim 4, **characterised in that** the inlet and outlet openings (12, 13) of the filter bag (7) are arranged in the vicinity of a top edge of the filter bag (7).

6. Method according to any of claims 1 to 5, **characterised in that** the filter bag (7) comprises a first and second sheet (14, 15) made from a flexible thermoplastic material bonded to one another around their periphery so as to form an interior volume, said bag (7) including a welding seam (16) between the sheets (14, 15) arranged to form the filtration compartment (8) and the storage compartment (10), said welding seam (16) being arranged to form said passage (11) between the compartments.

7. Method according to claim 6, **characterised in that** the passage (11) between the filtration compartment (8) and the storage compartment (10) is closed by welding the two sheets (14, 15).

8. Method according to claim 6 or 7, **characterised in that** the welding seam (16) comprises a fragile part so as to be able to separate the filtration compartment (8) and the storage compartment (10).

9. Method according to any of claims 1 to 8, **characterised in that** it further comprises the steps of:
- before the compression of the primary bag (2), constraining the storage compartment (10) of the filter bag (7) to a first volume, and
- after the extraction of the first blood component from the primary bag (2), constraining the storage compartment (10) of the filter bag (7) to a second volume.

10. Method according to any of claims 1 to 9, **characterised in that** it further comprises the step of:
- before the compression of the primary bag (2), constraining the filtration compartment (8) of the filter bag (7) to a third volume.

11. Filter bag for implementing the method according to any of claims 1 to 10, said filter bag (7) comprising a first and second sheet (14, 15) made from a flexible thermoplastic material bonded to one another around their periphery so as to form an interior volume, at least one inlet opening (12) and one outlet opening (13), and a welding seam (16) between the sheets (14, 15) arranged to form the filtration compartment (8) and the storage compartment (10), **characterised in that** said welding seam (16) comprises a horizontal part and a vertical part arranged to form with an edge of the filter bag (7) a channel (17) in fluidic communication with one of the inlet or outlet openings (12, 13) of the filter bag (7).

12. Apparatus for implementing the method according to any of claims 1 to 10, **characterised in that** it comprises:
- a means for compressing the first bag (2) so as to extract at least one blood component, and
- a control device for controlling the flow of fluids in the bag system (1), said control device comprising at least one clamp (46) for closing the passage (11) between the storage compartment (10) and the filtration compartment (8) .

13. Apparatus according to claim 12, **characterised in that** it comprises a receptacle (20) for receiving a bag system (1), said receptacle comprising a cavity (21) having a bottom (22) and a side wall (23), said cavity (21) being divided by a press plate (24) perpendicular to the bottom (22) of the cavity (21), into a first compartment (25) for receiving the first bag (2) and a secondary compartment (26) for receiving at least one satellite bag (3, 5) respectively, said press plate (24) being movable within said cavity (21) in a first direction towards said side wall (23) so as to compress the first bag (2).
